# EUROPÄISCHE PATENTSCHRIFT

(11) **EP 0 906 357 B1**
(45) Veröffentlichungstag und Bekanntmachung des Hinweises auf die Patenterteilung: **08.08.2001**
(21) Anmeldenummer: 97925006.5
(22) Anmeldetag: 28.05.1997
(51) Int. Cl.: C08G 69/48, C08F 290/14, B01J 20/32

(54) **POLYMERE SORBENTIEN AUF BASIS POLYMERISATIONSFÄHIGER DERIVATE VON POLYAMIDEN**
ABSORBENT POLYMERS BASED ON POLYMERISABLE POLYAMIDE DERIVATIVES
POLYMERES ABSORBANTS A BASE DE DERIVES POLYAMIDES POLYMERISABLES

(30) Priorität: 21.06.1996 DE 19624813; 06.07.1996 DE 19627404; 17.07.1996 DE 19628832; 19.07.1996 DE 19629206
(43) Veröffentlichungstag der Anmeldung: 07.04.1999
(73) Patentinhaber: MERCK PATENT GmbH, 64271 Darmstadt (DE)
(72) Erfinder: MÜLLER, Egbert, D-64390 Erzhausen (DE); HARDERS, Hans-Dieter, D-64287 Darmstadt (DE); LUBDA, Dieter, D-64625 Bensheim (DE)
(86) Internationale Anmeldenummer: EP9702768
(87) Internationale Veröffentlichungsnummer: WO9749754

(56) Entgegenhaltungen:
- EP-A- 0 143 037
- EP-A- 0 147 267
- DE-A- 4 129 901
- US-A- 4 563 507

## Beschreibung

Die Erfindung betrifft polymere Sorbentien auf Basis polymerisationsfähiger Derivate von Polyamiden sowie Formkörper, Affinitätsträger, immobilisierte Enzyme herstellbar aus den polymeren Sorbentien und Verfahren zur chromatographischen Stofftrennung unter Verwendung der polymeren Sorbentien.

In EP 0 147 267 und EP 0 143 037 werden mit polymerisierbaren Doppelbindungen derivatisierte Polyamide beschrieben. Sie werden beispielsweise durch Behandlung eines Polyamids mit einer amino- oder carboxylreaktiven Verbindung erzeugt.

Aus den Dokumenten DE 195 01 726 und WO 96/22 316 sind polymerisationsfähige Derivate von Polyamiden, sowie Verfahren zur Polymerisation auf derartig derivatisierten Polyamiden bekannt. Dabei werden folgende Reaktionsschritte ausgeführt:
1. Umsetzung der Aminogruppen eines Polyamids oder eines Formkörpers aus Polyamid mit einer Lösung einer Verbindung, die eine Doppelbindung enthält, wobei die Reaktion mit den Aminogruppen durch eine Oxirangruppe erfolgt; bei dieser Umsetzung entstehen polymerisierbare Derivate des Polyamids;
2. Polymerisieren von Monomeren an die im ersten Schritt eingefügten Doppelbindungen nach üblichen Verfahren, wobei ein Blockpolymer entsteht, das gegegebenfalls die mit den Monomeren eingeführten reaktiven Gruppen enthält, wobei insbesondere Oxirangruppen oder Gruppen, die sich in Azlactone überführen lassen, als reaktive Gruppen bevorzugt sind;
3. Optionale polymeranaloge Umsetzung der reaktiven Gruppen aus Schritt 2 zu einem Trennmaterial oder einem immobilisiertem Katalysator oder Enzym.

Verwendet man für diese Umsetzungen chirale Verbindungen, so sind chiral modifizierte Formkörper zugänglich, die für Enantiomerentrennungen geeignet sind.

Es besteht also die Aufgabe, weitere sorbentien auf Basis von insbesondere alkalistabilen, polymerisierbaren Derivaten von Polyamid oder Formkörpern aus Polyamid bereitzustellen.

Es wurde gefunden, daß anstelle der in DE 195 01 726 und WO 96/22 316 offenbarten Derivatisierungsmittel für den ersten Reaktionsschritt weitere Derivatisierungsmittel verwendet werden können. Dazu gehören auch Derivatisierungsmittel, die in analoger Weise die Umsetzung von Carboxylgruppen im Polyamid erlauben. Dabei zeigte sich, daß einige dieser Derivatisierungsmittel zu Derivaten führen, die eine verbesserte Stabilität in Gegenwart von alkalischen Lösungen aufweisen. Weiterhin wurde gefunden, daß die Dichte der polymerisierbaren Gruppen erhöht werden kann, wenn man im Basismaterial vorhandene Carboxylgruppen mit einem Diamin umsetzt. In analoger Weise kann die Dichte der polymerisierbaren Gruppen erhöht werden, indem man das Basispolymer (Polyamid) mit Dicarbonsäuren oder Dicarbonsäureanhydriden umsetzt. Anschließend werden die Carboxylgruppen mit einer Aminoverbindung, die ungesättigte Gruppen enthält, umgesetzt, und so polymerisierbare Gruppen eingeführt.

Gegenstand der Erfindung sind polymere Sorbentien, die durch die folgenden Reaktionsschritte erhältlich sind:
a) Einführung von polymerisierbaren Doppelbindungen durch Umsetzung der Kopplungsgruppen eines Polyamids, d.h. der Aminogruppen eines Polyamides mit einer aminoreaktiven Verbindung, die eine polymerisierbare Doppelbindung enthält, oder durch Umsetzung der Carboxylgruppen des Polyamides mit einer carboxylgruppenreaktiven Verbindung, die eine polymerisierbare Doppelbindung enthält, mit der Maßgabe, daß die genannte aminoreaktive Verbindung keinen Oxiranring enthält, wenn das Basispolymer umgesetzt wird, ohne zuvor mit einer Diaminoverbindung umgesetzt worden zu sein,
b) Polymerisation von Monomeren auf das nach Schritt a) derivatisierte Polyamid, wobei die genannten Monomere eine Epoxidgruppe, eine Azlactongruppe oder eine Vorläufergruppe für eine Azlactongruppe oder einen Separationseffektor enthalten,
c) optionale Umsetzung der genannten Epoxid- oder Azlactongruppe in einen Separationseffektor.

Erfindungsgemäß werden demnach unter dem Begriff "Kopplungsgruppen des Polyamides" die Amino- oder Carboxylgruppen des Polyamides verstanden. Bei den kommerziell üblichen Polyamiden stellen also die terminalen Amino- beziehungsweise Carboxylgruppen die Kopplungsgruppen dar. Unter dem Begriff "ethylenisch ungesättigtes Derivatisierungsmittel" werden erfindungsgemäß Reagenzien oder Kombinationen von Reagenzien verstanden, die mit den Kopplungsgruppen des Polyamides, d.h. mit den Carboxyl- oder mit den Aminogruppen, reagieren und dabei ethylenisch ungesättigte Gruppierungen in das entstehende Produkt einführen.

Beispiele für geeignete Derivatisierungsmittel, die mit Aminogruppen reagieren, sind in DE 195 01 726 und WO 96/22 316 offenbart. Deswegen sind mit polymerisierbaren Doppelbindungen derivatisierte Polyamide erhältlich durch Umsetzung von aminoreaktiven Verbindungen, die einen Oxiranring und eine ethylenisch ungesättigte Doppelbindung enthalten, nicht Gegenstand der Erfindung, soweit das Basispolymer umgesetzt wird, ohne zuvor mit einer Diaminoverbindung umgesetzt worden zu sein.

Neue geeignete Derivatisierungsmittel sind beispielsweise: Anhydride einer ethylenisch ungesättigten Carbonsäure, beispielsweise Methacrylsäureanhydrid oder -chlorid, Acrylsäureanhydrid oder -chlorid, oder Vinylazlactonderivate der Formel I, 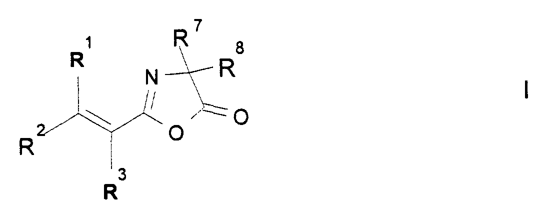 worin
- R¹, R² und R³: unabhängig voneinander
H oder CH₃;
- R⁷ und R⁸: unabhängig voneinander
H oder C₁- bis C₅- Alkyl
bedeuten.

Bevorzugt bedeuten R¹, R² und R³ H, sowie R⁷ und R⁸ Methyl.

Zu den ethylenisch ungesättigten Derivatisierungsmitteln gehören auch solche, die mit den Carboxylgruppen reagieren und auf diese Weise ethylenisch ungesättigte Gruppen einführen. Zu dieser Gruppe von ethylenisch ungesättigten Derivatisierungsmitteln gehört beispielsweise Allylamin, das beispielsweise durch Reaktion mit Carbodiimiden an Carboxylgruppen des Polyamids gebunden werden kann.

Es ist auch möglich, vor der Umsetzung mit dem ethylenisch ungesättigten Derivatisierungsmittel eine Umsetzung von Kopplungsgruppen des Polyamids mit einem bifunktionellen Derivatisierungsmittel auszuführen. So werden beispielsweise zusätzliche Aminogruppen in das Polyamid durch Umsetzung von Carboxylgruppen des Polyamides mit Diaminoverbindungen eingeführt. Umgekehrt ist es auch möglich, durch Umsetzung der Aminogruppen des Polyamides mit Dicarbonsäuren oder Dicarbonsäureanhydriden zusätzliche Carboxygruppen einzuführen.

Besonders bevorzugt sind polymere Sorbentien, bei denen in Schritt b) Acrylsäure als Monomer oder Monomereinheiten der Formel II, III, IV oder V verwendet werden:

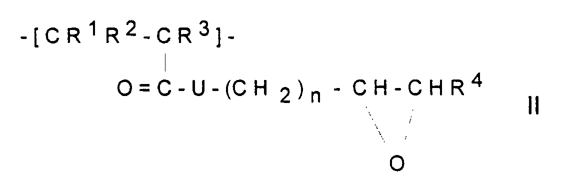

In Formel II bedeuten:
- R¹,R² und R³: unabhängig voneinander
H oder CH₃,
- R⁴: H, Alkyl mit 1-5 C-Atomen oder Aryl mit 6-12 C-Atomen,
- U: - O - oder - NH -
und
- n: eine ganzen Zahl zwischen 1 und 5.

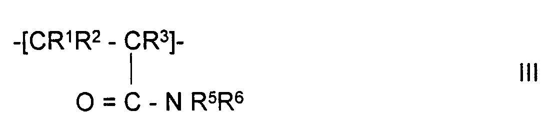

In Formel III bedeuten:
- R¹,R² und R³: unabhängig voneinander
H oder CH₃,
- R⁵: H, mit -COOH, mit -SO₃H, mit -NR⁷R⁸ oder mit -N⁺R⁷R⁸R⁹ substituiertes Alkyl mit 1-5 C-Atomen oder mit -COOH, -SO₃H, mit -NR⁷R⁸ oder mit -N⁺R⁷R⁸R⁹ substituiertes Aryl mit 6-12 C-Atomen,
- R⁶: mit -COOH, mit -SO₃H, mit -NR⁷R⁸ oder mit -N⁺R⁷R⁸R⁹ substituiertes Alkyl mit 1-5 C-Atomen oder mit -COOH, -SO₃H, mit -NR⁷R⁸ oder mit -N⁺R⁷R⁸R⁹ substituiertes Aryl mit 6-12 C-Atomen,
wobei
- R⁵ und R⁶: so abgestimmt sind, daß entweder beide Reste sauer oder basisch oder einer der Reste neutral ist,
- R⁷ und R⁸: unabhängig voneinander
H, Alkyl mit 1-5 C-Atomen
und
- R⁹: Alkyl mit 1-5 C-Atomen.

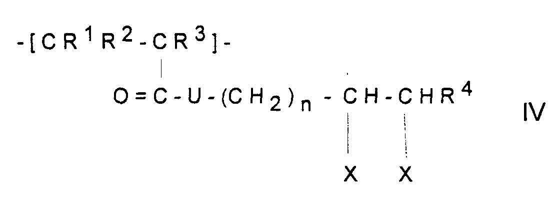

In Formel IV bedeuten:
- R¹,R² und R³: unabhängig voneinander
H oder CH₃,
- R⁴: H, Alkyl mit 1-5 C-Atomen oder Aryl mit 6-12 C-Atomen,
- U: - O - oder - NH -
- n: eine ganzen Zahl zwischen 1 und 5,
ein Rest X einen Separationseffektor, sowie der andere Rest X OH.

Der Separationseffektor kann insbesondere eine der folgenden Bedeutungen aufweisen:
a) eine ionische Gruppe, ausgewählt aus -PO₄H₂, -SO₃H, -NR⁷R⁸ oder -N+R⁷R⁸R⁹,
   worin
   - R⁷ und R⁸: unabhängig voneinander
   H, Alkyl mit 1-5 C-Atomen
   und
   - R⁹: Alkyl mit 1-5 C-Atomen
   mit der Maßgabe, daß wenn X = -N⁺R⁷R⁸R⁹, R⁷ und R⁸ nicht H sein können,
b) eine hydrophobe Gruppierung -OR¹⁰ oder -NHR¹⁰, wobei R¹⁰ C₁-C₂₀-Alkyl, C₆-C₂₅-Aryl, C₇-C₂₅-Alkylaryl oder C₇-C₂₅-Arylalkyl bedeuten, und wobei diese Reste auch mit Nitril oder C₁-C₅-Alkoxy derivatisiert sein können, und wobei auch eine oder mehrere nicht benachbarte CH₂-Gruppen durch NH oder O oder auch eine oder mehrere CH Gruppen durch N ersetzt sein können;
c) eine Metallchelat-Gruppierung;
d) ein thiophiler Rest;
e) ein chiraler Rest.

Thiophile Reste sind beispielsweise in EP 0 165 912 offenbart.

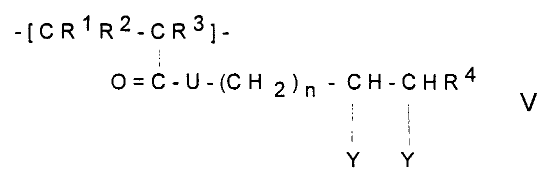

In Formel V bedeuten:
- R¹,R² und R³: unabhängig voneinander
H oder CH₃,
- R⁴: H, Alkyl mit 1-5 C-Atomen oder Aryl mit 6-12 C-Atomen, n eine ganzen Zahl zwischen 1 und 5,
- U: -O- oder -NH-
ein Rest Y einen Rest der Formel VI und der andere Rest Y OH 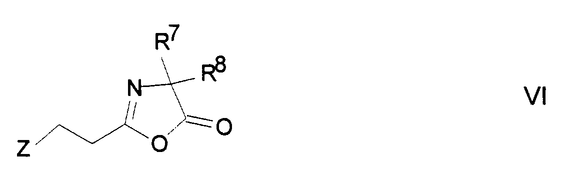
- Z: -S oder - NH
und
- R⁷ und R⁸: unabhängig voneinander
H oder Alkyl mit 1-5 C-Atomen.

Insbesondere die polymeren Sorbentien, die Monomereinheiten der Formel II oder V enthalten, können für die Herstellung von Affinitätsträgern oder zur Immobilisierung von Enzymen verwendet werden.

Gegenstand der Erfindung sind somit auch Affinitätsträger und immobilisierte Enzyme herstellbar aus einem erfindungsgemäßen polymeren Sorbens.

Es sind poröse und unporöse Formkörper aus Polyamiden bekannt; dazu gehören beispielsweise Membranen, Schwämme, Schläuche und Hohlfasermembranen. Gegenstand der Erfindung sind somit auch derartige Formkörper, die im wesentlichen aus einem erfindungsgemäßen polymermodifizierten Material bestehen, und die zusätzlich auch Affinitätsliganden oder immobilierte Enzyme enthalten können.

Ferner ist Gegenstand der Erfindung ein Verfahren zu chromatograpischen Stofftrennung, bei dem ein erfindungsgemäßes polymeres Sorbens verwendet wird.

Erfindungsgemäß werden bei dem ersten Reaktionsschritt ungesättigte Reste in das Polyamid eingeführt. Dazu können neben den aus DE 195 01 726 bekannten Reaktionsfolgen die im weiteren offenbarten Reaktionsfolgen dienen. Gemeinsam ist diesen Reaktionsfolgen, daß ein ethylenisch ungesättigtes Derivatisierungsmittel, d.h. eine amino- oder carboxyreaktive Verbindung, die eine polymerisierbare Doppelbindung enthält, mit den Amino- beziehungsweise Carboxylgruppen des Polyamids zur Reaktion gebracht werden. Aminoreaktive Verbindungen sind grundsätzlich dem Fachmann in großer Zahl bekannt: Dazu gehören beispielsweise Oxiranderivate, Säureanhydride, Säureazide, Azlactonderivate. In ähnlicher Weise können Reaktionsfolgen verwendet werden, die bei Peptidsynthesen Verwendung finden, beispielsweise Reaktionen mit wasserabspaltenden Mitteln (z.B. Carbodiimiden) oder die Verwendung von aktivierten Estern (z.B. p-Nitrophenylester). In analoger Weise ist es möglich, die freien Carboxylgruppen von Polyamiden zu derivatisieren, so daß diese ebenfalls als Ansatzpunkte für eine Polymerisation zur Verfügung stehen. Dazu können Verbindungen, die mit Carboxylgruppen reagieren können, und die polymerisierbare Doppelbindungen enthalten, z.B. Allylamin, verwendet werden.

Soweit das Basispolymer sowohl Carboxyl- als auch Aminogruppen enthält, ist es möglich, die Beladungsdichte zu erhöhen, indem vor der Umsetzung mit dem ethylenisch ungesättigten Derivatisierungsmittel eine Umsetzung mit einem bifunktionellen Derivatisierungsmittel erfolgt, d.h. mit einer Diaminoverbindung oder mit einer Dicarbonsäure oder einem Dicarbonsäureanhydrid. So können beispielsweise durch die Umsetzung mit 1,2-Ethylendiamin zusätzliche Aminogruppen in das Basispolymer eingeführt werden, die wie oben beschrieben weiter umgesetzt werden können. Als Diaminoverbindungen sind insbesondere α,ω-Diaminoalkane, wie zum Beispiel 1,2-Ethylendiamin, 1,3-Diaminopropan, 1,4-Diaminobutan oder 1,6-Diaminohexan bevorzugt. Es ist ebenfalls möglich, die Aminogruppen des Polyamids mit einer Dicarbonsäure oder einem Dicarbonsäureanhydrid umzusetzen, um anschließend an den Carboxylgruppen mit Aminoverbindungen, die polymerisierbare Doppelbindungen enthalten, z.B. Allylamin, polymerisierbare Doppelbindungen einzufügen.

Reaktionsfolgen für die genannten Umsetzungen sind dem Fachmann bekannt, dazu gehören insbesonders auch Umsetzungen mit wasserabspaltenden Mitteln, oder Umsetzungen, bei denen einer der Reaktionspartner in aktivierter Form vorliegt. Beispielsweise wird die Reaktion unter Verwendung von Carbodiimiden oder durch Aktivierung der Carboxylgruppen als Säureazid ausgeführt. Bevorzugt ist dabei die Umsetzung mit wasserlöslichen Carbodiimiden, wie z.B. 1-Ethyl-3-(3-dimethylaminopropyl)-carbodiimid (EDC), in wäßrigem oder organischwäßrigen Lösungen, die bevorzugt gepuffert sind. Als Puffersubstanzen werden Natriumacetat oder Morpholinethansulfonsäure bevorzugt. Bei derartigen Reaktionen beträgt der pH üblicherweise zwischen 4 und 6.

Als Basispolymer geeignetete Polyamide sind dem Fachmann bekannt und sind auch kommerziell erhältlich. Dazu gehören z.B. die unter dem Handelsnamen NYLON® bekannten Polymere, z.B. NYLON® 66 und NYLON® 6. Poröse oder unporöse Formkörper bestehend aus derartigen Polyamiden sind ebenfalls bekannt und auch kommerziell erhältlich; dazu gehören beispielsweise perlförmige Formkörper, Membranen, Schläuche, Hohlfasermembranen, Schwämme. Die Umsetzung derartiger Formkörper ist bevorzugt, da unter den Reaktionsbedingungungen, wie sie in DE 195 01 726 verwendet werden (Reaktionstemperatur unter 60 °C), deren Form erhalten bleibt, während andere Verfahren zur Derivatisierung von Polyamid in der Schmelze oder in Lösung ausgeführt werden.

Die Umsetzung mit den Anhydriden von ethylenisch ungesättigten Carbonsäuren erfolgt nach bekannten Verfahren, beispielsweise dem Schotten-Baumann-Verfahren bei Temperaturen unter 60 °C; dabei entstehen polymerisationsfähige Derivate von Polyamiden. Erfindungsgemäß werden unter dem Begriff Anhydride von ethylenisch ungesättigten Carbonsäuren sowohl die einfachen als auch die gemischten Anhydride verstanden. Zur ersten Gruppe gehören beispielsweise Acrylsäureanhydrid und Methacrylsäureanhydrid, zur zweiten Gruppe gehören beispielsweise Säurechloride wie Acrylsäurechlorid und Methacrylsäurechlorid. Weitere Beispiele für ethylenisch ungesättigte Carbonsäuren sind Crotonsäure, Isocrotonsäure, Vinylessigsäure und auch Sorbinsäure. Bevorzugte Anhydride von ethylenisch ungesättigten Carbonsäuren sind Acrylsäurechlorid, Methacrylsäurechlorid, Acrylsäureanhydrid und Methacrylsäureanhydrid.

Die Umsetzung der Aminogruppen des Polyamides mit einem Vinylazlactonderivat der Formel I, 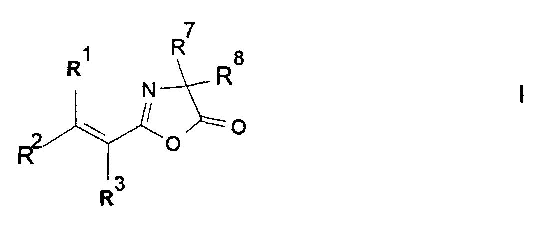 worin
- R¹, R² und R³: unabhängig voneinander
H oder CH₃;
- R⁷ und R⁸: unabhängig voneinander
H oder C₁- bis C₅- Alkyl
bedeuten,
erfolgt nach dem Fachmann bekannten Verfahren. Bevorzugt bedeuten R¹, R² und R³ H, sowie R⁷ und R⁸ Methyl; derartige Vinylazlactonderivate sind kommerziell erhältlich. Weitere Vinylazlactonderivate der Formel I sind ebenfalls kommerziell erhältlich oder nach Standardverfahren zugänglich.

Die üblicherweise verwendeten Polyamide, wie z.B. NYLON® 66 oder NYLON® 6 enthalten nur endständige freie Carboxyl- und/oder Aminogruppen. In diesem Fall entsteht bei der Polymerisation mit Monomeren auf das derivatisierte Basispolymer ein Blockpolymerisat. Falls das Basispolymer neben den endständigen freien Carboxyl- und/oder Aminogruppen noch seitenständige freie Carboxyl- und/oder Aminogruppen enthält, so entstehen zusätzlich seitenständige polymerisierbare Gruppen. Bei einer anschließenden Polymerisation findet dann zusätzlich zur Bildung des Blockpolymers eine Pfropfung statt. Block- und Pfropfpolymerisate werden zusammengefaßt erfindungsgemäß als polymermodifizierte Basispolymere oder polymermodifizierte Materialien bezeichnet.

Durch die Umsetzung des Polyamids, wie sie in der vorliegenden Anmeldung offenbart wird, werden ähnlich wie in DE 195 01 726 ungesättigte C=C-Gruppen in das Polyamid eingeführt. An diese Gruppen können weitere Monomere nach allgemein bekannten Verfahren polymerisiert werden, wobei ein erfindungsgemäßes polymermodifiziertes Basispolymer entsteht. Die Auswahl dieser Monomeren richtet sich nach dem vorgesehenen Verwendungszweck der derivatisierten Membran:
a) Aus DE 38 11 042 (EP 0 337 144; US 5,453,186) sind unter anderem Monomere bekannt, die zur Herstellung von lonenaustauschern geeignet sind; dazu gehören beispielsweise Acrylsäure, N-(Sulfoethyl)-acrylamid, 2-Acrylamldo-2-methylpropansulfonsäure, N,N-Dimethylaminoethyl-acrylamid, N,N-Diethylaminoethyl-acrylamid, sowie Trimethylammoniumethylacrylamid.
   Andere in dieser Druckschrift genannte Monomere erlauben die Bindung von Affinitätsliganden oder von Enzymen, oder eignen sich für reversed-phase Chromatographie: dazu gehören beispielsweise Acrylsäure, Acrylamid, Allylamin oder Acrylnitril.
b) Aus DE 43 10 964 (EP 0 565 978) sind Monomere bekannt, die einen Oxiranring, einen Azlactonring oder eine Gruppierung enthalten, die in einen Azlactonring umgesetzt werden kann. Polymere, die derartige Monomere enthalten, sind besonders gut für die Bindung von Affinitätsliganden oder von Enzymen geeignet. Affinitätsliganden sind beispielhaft in DE 43 10 964 offenbart.
   Aus DE 43 34 359 (WO 95/10 354) sind außerdem Verfahren zur Herstellung von Polymeren bekannt, die sich vom Poly(meth)acrylamiden ableiten, und deren Amidgruppen einen Oxiranring aufweisen.
c) Aus EP 0 249 078, EP 0202 770 und EP 0 448 823 sind optisch aktive Monomere bekannt, die sich auf die mit polymerisierbaren Doppelbindungen derivatisierten Basispolymere aufpolymerisieren lassen. Es resultieren somit polymermodifizierte Basispolymere, die als chirale Sorbentien verwendbar sind.
   Weiterhin können die Epoxid- oder Azlactongruppen in derartigen Polymeren in vorteilhafter Weise weiter umgesetzt werden, wodurch lonenaustauscher, thiophile Sorbentien oder Sorbentien für die Metallchelat- oder die hydrophobe Chromatographie, sowie auch für chirale Trennungen bereitgestellt werden. Dabei werden beispielsweise Phosphorsäure, Ammoniak, Diethylamin, Trimethylamin, schweflige Säure oder auch Komplexbildner wie Iminodiessigsäure, oder chirale Verbindungen, wie Proteine, Peptide oder Polysaccharidderivate, an den Oxiranring oder die Azlactongruppe addiert; Beispiele für derartige polymeranaloge Umsetzungen sind:

a) Die Herstellung von thiophilen Sorbentien und von Sorbenzien für die Metallchelatchromatographie ist in DE 43 10 964 (EP 0 565 978) offenbart.
b) In DE 43 33 674 (WO 95/09 964) und in DE 43 33 821 (WO95/09 695) sind derartige Umsetzungen, mit derer Hilfe lonenaustauscher bereitgestellt werden können, offenbart.
c) In DE 43 23 913 (WO 95/02 820) werden Sorbenzien für die hydrophobe Interaktionschromatographie beschrieben.

Erfindungsgemäß werden die Gruppen, die nach den oben genannten Verfahren in den chromatographischen Träger eingeführt werden, und die für die Trennung der Analyte wesentlich sind, zusammenfassend als Separationseffektoren bezeichnet.

Einzelheiten der Herstellung der verschiedenen Sorbenzien und deren Verwendung können den oben genannten Druckschriften entnommen werden.

Die Reaktion, bei der weitere Monomere auf das erfindungsgemäß derivatisierte Polyamid aufpolymerisiert werden, kann wie in DE 195 01 726 offenbart ausgeführt werden. Entsprechend können die erfindungsgemäß derivatisierten und polymermodifizierten Membranen, die an den aufpolymerisierten Ketten Separationseffektoren enthalten, für Stofftrennungen in ähnlicher Weise eingesetzt werden, wie es beispielsweise für partikuläre Sorbenzien mit ähnlichen Separationseffektoren üblich ist. Bezüglich der Herstellung und Verwendung von polymermodifizierten Polyamid-Derivaten wird auf die Offenbarung der Druckschriften DE 195 01 726 und WO 96/22 316 verwiesen.

Die folgenden Beispiele sollen den Gegenstand der Erfindung näher erläutern; sie stellen keine Einschränkung des Erfindungsgegenstandes dar.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

### Beispiele

Im folgenden wird unter Raumtemperatur eine Temperatur zwischen 15 und 30 °C verstanden.

### Beispiel 1: Reaktion eines Polyamids mit Ethylendiamin

Zur Durchführung der Synthese wird ein Polyamidhohlfaserbündel aus Polyamid 6 in eine 300-10 mm Chromatographiesäule SUPERFORMANCE® (Fa. Merck KGaA) gepackt. An diese Säule wird eine inerte Pumpe angeschlossen. Für die Umsetzung werden 10 Mol Ethylendiamin und 0,2 Mol EDC in 200 ml 0,1 M Natriumacetatpuffer (pH 4,7) gelöst und mit hoher Geschwindigkeit (5 ml/min) bei Raumtemperatur 5 Stunden im Kreis gepumpt. Anschließend wird die derivatisierte Membran 1 M Phosphatpuffer pH 7 und mit Wasser neutral gewaschen.

Das Ausgangsmaterial weist einen Gehalt an Aminogruppen von 1,2 µmol/g auf; nach der Reaktion beträgt der Gehalt an Aminogruppen von 12,3 µmol/g.

### Beispiel 2: Einführung von C=C-Bindungen in ein amino-derivatisiertes Polyamid mit Acrylsäurechlorid

In der in Beispiel 1 beschriebenen Apparatur wird die nach Beispiel 1 aminoderivatisierte Membran mit 200 ml einer wäßrigen Lösung (1 M Acrylsäurechlorid in 1 M NaOH) behandelt (Umpumpen der Lösung mit 5 ml/min; eine Stunde bei 4 °C). Anschließend wird die derivatisierte Hohlfasermembran mit 1 M Natriumphosphatpuffer pH 7 und Wasser neutral gewaschen.

### Beispiel 3: Einführung von C=C-Bindungen in ein Polyamid mit Acrylsäurechlorid

In der in Beispiel 1 beschriebenen Apparatur wird ein Polyamidhohlfaserbündel aus NYLON® mit 200 ml einer wäßrigen Lösung (1 M Acrylsäurechlorid in 1 M NaOH) behandelt (Umpumpen der Lösung mit 5 ml/min; eine Stunde bei 4 °C). Anschließend wird die derivatisierte Hohlfasermembran mit 1 M Natriumphosphatpuffer pH 7 und Wasser neutral gewaschen.

### Beispiel 4: Einführung von C=C-Bindungen in ein Polyamid mit Vinyldimethylazlacton

In der in Beispiel 1 beschriebenen Apparatur wird ein Polyamidhohlfaserbündel aus NYLON® mit einer Lösung aus 100 ml Vinyldimethylaziacton in 100 ml Aceton bei Raumtemperatur behandelt. Anschließend wird mit Aceton und Toluol gespült.

### Beispiel 5: Einführung von C=C-Bindungen in ein Polyamid (Reaktion mit Allylamin)

Zur Durchführung der Synthese wird ein Polyamidhohlfaserbündel aus Polyamid 6 in eine 300-10 mm Chromatographiesäule SUPERFORMANCE® (Fa. Merck KGaA) gepackt. An diese Säule wird eine inerte Pumpe angeschlossen. Für die Umsetzung werden 10 Mol Allylamin und 0,2 Mol EDC in 200 ml 0,1 M Natriumacetatpuffer (pH 4,7) gelöst und mit hoher Geschwindigkeit (5 ml/min) bei Raumtemperatur 5 Stunden im Kreis gepumpt. Anschließend wird die derivatisierte Membran 1 M Phosphatpuffer pH 7 und mit Wasser neutral gewaschen.

Aus nach den Beispielen 2 bis 5 derivatisierten Membranen, können nach den in DE 195 01 726.9 offenbarten Verfahren Blockpolymere hergestellt werden. Die folgenden Beispiele verdeutlichen diese Reaktion.

### Beispiel 6: Blockpolymerisat mit Monomereinheiten aus Glycidylmethacrylat

Die nach Beispiel 3 derivatisierte Hohlfasermembran wird in der dort beschriebenen Apparatur mit zunächst mit Aceton, dann mit Toluol (jeweils 200 ml) gespült. Anschließend wird eine Lösung von 15 g Glycidylmethacrylat und 1 g Azoisobutyronitril (Polymerisationsinitiator) in 200 ml Toluol bei 100 °C eine Stunde umgepumpt (Fluß: 2 ml/min). Die derivatisierte Hohlfasermembran wird anschließend mit Toluol, Aceton und Wasser gespült.

### Beispiel 7: Blockpolymerisat mit Monomereinheiten eines Chlorhydrinderivates des Acrylamids und polymeranaloge Umsetzung zum Oxiranderivat

Statt Glycidylmethacrylat wird das Chlorhydrinderivat des Acrylamids auf die nach Beispiel 4 derivatisierte Membran aufpolymerisiert. Durch Behandlung mit 1 M Natronlauge bei 60 °C (5 Stunden) wird das Oxiranderivat hergestellt. Nach dem Abkühlen wird neutral gewaschen.

Einzelheiten der Reaktionsfolge sind in DE 43 34 359 offenbart.

Aus Blockpolymeren, die nach den obigen Beispielen 6 und 7 hergestellt werden, können nach den in DE 195 01 726 offenbarten Verfahren durch polymeranaloge Reaktion nach an sich bekannten Verfahren Trennmaterialien oder immobilisierte Enzyme oder immobilisierte Katalysatoren hergestellt werden. Das folgende Beispiel verdeutlicht eine solche Reaktion.

### Beispiel 8: Erzeugung eines Anionenaustauschers vom SO₃⁻- Typ

In 200 ml Wasser werden 10 g Natriumdihydrogenphoshat, 40 g Natriumsulfit und 10 g Tetrabutylammoniumhydrogensulfat gelöst und die Lösung auf pH 8 eingestellt. In der in den obigen Beispielen beschriebenen Apparatur wird das Hohlfaserbündel, auf das nach Beispiel 7 Epoxypropylmethacrylat polymerisiert wurde, mit der wäßrigen Lösung behandelt (2,5 Stunden, 95 °C, Fluß: 5 ml/min). Anschließend wird mit Wasser, 1 M Natronlauge, Wasser, 1 M Phosphatpuffer, pH 7 und mit Wasser gewaschen.

## Patentansprüche

1. Polymeres Sorbens erhältlich durch folgende Reaktionsschritte:
a) Einführung von polymerisierbaren Doppelbindungen durch Umsetzung der Aminogruppen eines Polyamides mit einer aminoreaktiven Verbindung, die eine polymerisierbare Doppelbindung enthält, oder durch Umsetzung der Carboxylgruppen des Polyamides mit einer carboxylgruppenreaktiven Verbindung, die eine polymerisierbare Doppelbindung enthält, mit der Maßgabe, daß die genannte aminoreaktive Verbindung keinen Oxiranring enthält, wenn das Basispolymer umgesetzt wird, ohne zuvor mit einer Diaminoverbindung umgesetzt worden zu sein,
b) Polymerisation von Monomeren auf das nach Schritt a) derivatisierte Polyamid, wobei die genannten Monomere eine Epoxidgruppe, eine Azlactongruppe oder eine Vorläufergruppe für eine Azlactongruppe oder einen Separationseffektor enthalten,
c) optionale Umsetzung der genannten Epoxid- oder Azlactongruppe in einen Separationseffektor.

2. Polymeres Sorbens nach Anspruch 1, dadurch gekennzeichnet, daß vor der Umsetzung mit der genannten aminoreaktiven Verbindung in Schritt a) Carboxylgruppen des Polymers mit einer Diaminoverbindung umgesetzt werden.

3. Polymeres Sorbens nach Anspruch 1, dadurch gekennzeichnet, daß vor der Umsetzung mit der genannten carboxylgruppenhaltigen Verbindung in Schritt a) Aminogruppen des Polymers mit einer Dicarbonsäure oder einem Dicarbonsäureanhydrid umgesetzt werden.

4. Polymeres Sorbens nach Anspruch 1 oder 2 erhältlich, indem in Schritt a) die Aminogruppen des Polyamides mit einem Anhydrid einer ethylenisch ungesättigten Carbonsäure umgesetzt werden.

5. Polymeres Sorbens nach Anspruch 1 oder 2, wobei in Schritt a) die Aminogruppen des Polyamides mit einem Vinylazlactonderivat der Formel I umgesetzt werden, 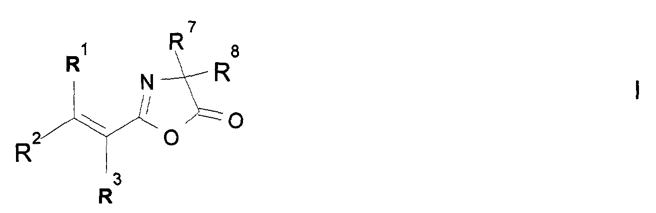 worin
R¹, R² und R³ unabhängig voneinander
H oder CH₃;
R⁷ und R⁸ unabhängig voneinander
H oder C₁- bis C₅- Alkyl
bedeuten.

6. Polymeres Sorbens nach einem der Ansprüche 1 bis 5, dadurch gekennzeichnet, daß als Monomer in Reaktionsschritt b) Acrylsäure verwendet wird.

7. Polymeres Sorbens nach einem der Ansprüche 1 bis 5, dadurch gekennzeichnet, daß das aufpolymerisierte Polymer Monomereinheiten der Formel II, 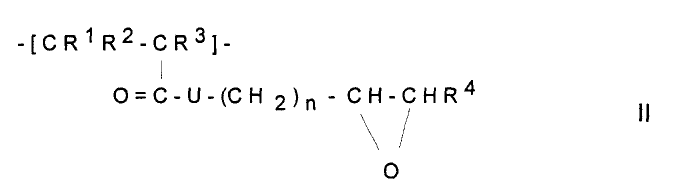 worin
R¹,R² und R³ unabhängig voneinander
H oder CH₃,
R⁴ H, Alkyl mit 1-5 C-Atomen oder Aryl mit 6-12 C-Atomen,
U - O - oder - NH -
und
n eine ganzen Zahl zwischen 1 und 5
bedeuten, enthält.

8. Polymeres Sorbens nach einem der Ansprüche 1 bis 5, dadurch gekennzeichnet, daß das aufpolymerisierte Polymer Monomereinheiten der Formel V, 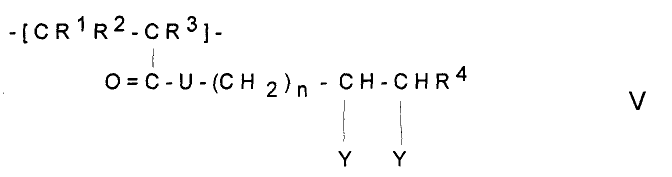 worin
R¹,R² und R³ unabhängig voneinander
H oder CH₃,
R⁴ H, Alkyl mit 1-5 C-Atomen oder Aryl mit 6-12 C-Atomen,
U -O- oder - NH-,
n eine ganzen Zahl zwischen 1 und 5,
ein Rest Y einen Rest der Formel VI und der andere Rest Y OH 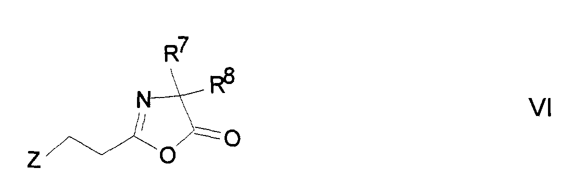
Z -S oder -NH
und
R⁷ und R⁸ unabhängig voneinander
H oder Alkyl mit 1-5 C-Atomen
bedeuten.

9. Polymeres Sorbens nach einem der Ansprüche 1 bis 5, dadurch gekennzeichnet, daß das aufpolymerisierte Polymer Monomereinheiten der Formel III, 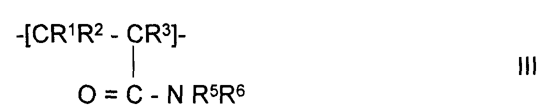 worin
R¹,R² und R³ unabhängig voneinander
H oder CH₃,
R⁵ H, mit -COOH, mit -SO₃H, mit -NR⁷R⁸ oder mit -N⁺R⁷R⁸R⁹ substituiertes Alkyl mit 1-5 C-Atomen oder mit -COOH, -SO₃H, mit -NR⁷R⁸ oder mit -N⁺R⁷R⁸R⁹ substituiertes Aryl mit 6-12 C-Atomen,
R⁶ mit -COOH, mit -SO₃H, mit -NR⁷R⁸ oder mit -N⁺R⁷R⁸R⁹ substituiertes Alkyl mit 1-5 C-Atomen oder mit -COOH, -SO₃H, mit -NR⁷R⁸ oder mit -N⁺R⁷R⁸R⁹ substituiertes Aryl mit 6-12 C-Atomen,
wobei R⁵ und R⁶ so abgestimmt sind, daß entweder beide Reste sauer oder basisch oder einer der Reste neutral ist,
R⁷ und R⁸ unabhängig voneinander
H, Alkyl mit 1-5 C-Atomen
und
R⁹ Alkyl mit 1-5 C-Atomen
bedeuten, enthält.

10. Polymeres Sorbens nach einem der Ansprüche 1 bis 5, dadurch gekennzeichnet, daß das aufpolymerisierte Polymer Monomereinheiten der Formel IV, 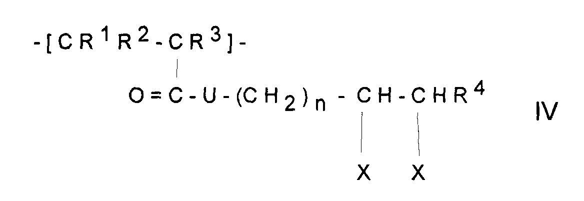 worin
R¹,R² und R³ unabhängig voneinander
H oder CH₃,
R⁴ H, Alkyl mit 1-5 C-Atomen oder Aryl mit 6-12 C-Atomen,
U -O- oder -NH-,
n eine ganzen Zahl zwischen 1 und 5,
ein Rest X einen Separationseffektor, sowie der andere Rest X OH
bedeuten, enthält.

11. Polymeres Sorbens nach Anspruch 10, dadurch gekennzeichnet, daß der Separationseffektor eine ionische Gruppe, ausgewählt aus -PO₄H₂, -SO₃H, -NR⁷R⁸ oder -N+R⁷R⁸R⁹, darstellt,
worin
R⁷ und R⁸ unabhängig voneinander
H, Alkyl mit 1-5 C-Atomen
und
R⁹ Alkyl mit 1-5 C-Atomen
mit der Maßgabe, daß wenn X = -N⁺R⁷R⁸R⁹, R⁷ und R⁸ nicht H sein können,
bedeutet.

12. Polymeres Sorbens nach Anspruch 10, dadurch gekennzeichnet, daß der Separationseffektor eine hydrophobe Gruppierung -OR¹⁰ oder -NHR¹⁰,
wobei
R¹⁰ C₁-C₂₀-Alkyl, C₆-C₂₅-Aryl, C₇-C₂₅-Alkylaryl oder C₇-C₂₅-Arylalkyl bedeuten, und wobei diese Reste auch mit Nitril oder C₁-C₅-Alkoxy derivatisiert sein können, und wobei auch eine oder mehrere nicht benachbarte CH₂-Gruppen durch NH oder O oder auch eine oder mehrere CH Gruppen durch N ersetzt sein können,
bedeutet.

13. Polymeres Sorbens nach Anspruch 10, dadurch gekennzeichnet, daß der Separationseffektor eine Metallchelat-Affinitätsgruppe darstellt.

14. Polymeres Sorbens nach Anspruch 10, dadurch gekennzeichnet, daß der Separationseffektor einen thiophilen Rest darstellt.

15. Affinitätsträger herstellbar aus einem polymeren Sorbens nach einem der Ansprüche 1-9 oder 11.

16. Immobilisiertes Enzym herstellbar aus einem polymeren Sorbens nach einem der Ansprüche 1-9 oder 11.

17. Formkörper im wesentlichen bestehend aus einem polymeren Sorbens nach einem der Ansprüche 1 bis 14.

18. Formkörper, an dem ein Affinitätsligand oder ein Enzym immobilisiert ist, herstellbar aus einem polymeren Sorbens nach einem der Ansprüche 1 bis 9 oder 11.

19. Verfahren zur chromatograpischen Stofftrennung, dadurch gekennzeichnet, daß ein polymeres Sorbens nach einem der Ansprüche 1 bis 15 verwendet wird.

## Claims

1. Polymeric sorbent obtainable by the following reaction steps: a) introduction of polymerizable double bonds by reaction of the amino groups of a polyamide with an amino-reactive compound which contains a polymerizable double bond, or by reaction of the carboxyl groups of the polyamide with a carboxyl group-reactive compound which contains a polymerizable double bond, with the proviso that the amino-reactive compound mentioned contains no oxirane ring if the base polymer is reacted without having been reacted beforehand with a diamino compound.
b) polymerization of monomers onto the polyamide derivatized according to step a), the monomers mentioned containing an epoxide group, an azlactone group or a precursor group of an azlactone group, or a separation effector,
c) optional conversion of the epoxide or azlactone groups mentioned into a separation effector.

2. Polymeric sorbent according to Claim 1, characterized in that carboxyl groups of the polymer are reacted with a diamino compound before the reaction in step a) with the amino-reactive compound mentioned.

3. Polymeric sorbent according to Claim 1, characterized in that amino groups of the polymer are reacted with a dicarboxylic acid or a dicarboxylic acid anhydride before the reaction in step a) with the carboxyl group-containing compound mentioned.

4. Polymeric sorbent according to Claim 1 or 2, obtainable by reaction of the amino groups of the polyamide with an anhydride of an ethylenically unsaturated carboxylic acid in step a).

5. Polymeric sorbent according to Claim 1 or 2, where of the amino groups of the polyamide are reacted in step a) with a vinylazlactone derivative of the formula I 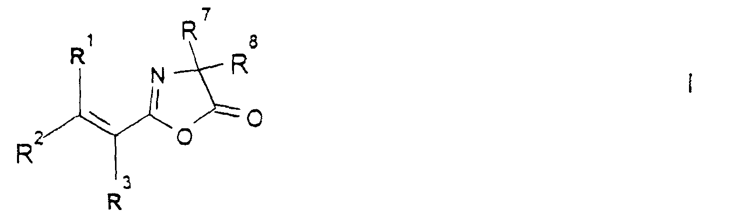 wherein
R¹, R² and R³ independently of one another are
H or CH₃;
R⁷ and R⁸ independently of one another are
H or C₁ - to C₅-alkyl.

6. Polymeric adsorbent according to one of Claims 1 to 5, characterized in that the monomer used in reaction step b) is acrylic acid.

7. Polymeric sorbent according to one of Claims 1 to 5, characterized in that the polymerized-on polymer contains monomer units of the formula II, 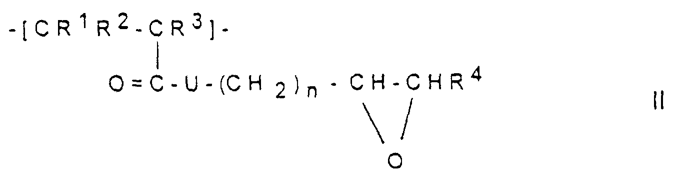 wherein
R¹, R² and R³ independently of one another are
H or CH₃,
R⁴ is H, alkyl having 1-5 C atoms or aryl having 6-12 C atoms,
U is -O- or -NH-
and
n is an integer between 1 and 5.

8. Polymeric sorbent according to one of Claims 1 to 5, characterized in that the polymerized-on polymer contains monomer units of the formula V 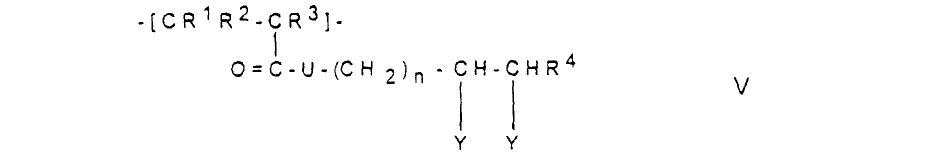 wherein
R¹, R² and R³ independently of one another are
H or CH₃,
R⁴ is H, alkyl having 1-5 C atoms or aryl having 6-12 C atoms,
U is -O- or -NH-,
n is an integer between 1 and 5,
one radical Y is a radical of the formula VI and the other radical Y is OH 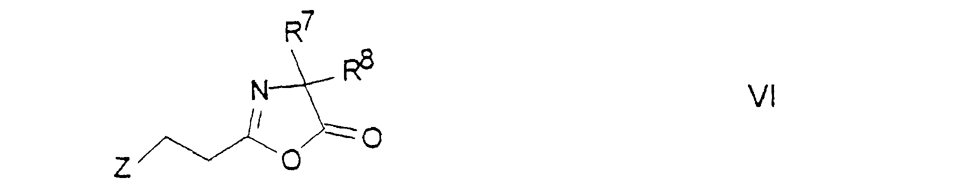
Z is -S or -NH
and
R⁷ and R⁸ independently of one another are H or alkyl having 1-5 C atoms.

9. Polymeric sorbent according to one of Claims 1 to 5, characterized in that the polymerized-on polymer contains monomer units of the formula III 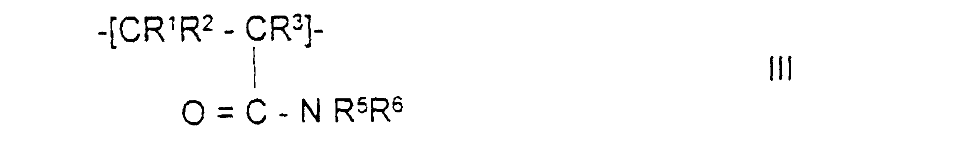 wherein
R¹, R² and R³ independently of one another are
H or CH₃,
R⁵ is H, alkyl having 1-5 C atoms which is substituted by -COOH, by -SO₃H, by -NR⁷R⁸ or by -N⁺R⁷R⁸R⁹, or aryl having 6-12 C atoms which is substituted by -COOH, by -SO₃H, by -NR⁷R⁸ or by -N⁺R⁷R⁸R⁹,
R⁶ is alkyl having 1-5 C atoms which is substituted by -COOH, by -SO₃H, by -NR⁷R⁸ or by -N⁺R⁷R⁸R⁹, or aryl having 6-12 C atoms which is substituted by -COOH, by -SO₃H, by -NR⁷R⁸ or by -N⁺R⁷R⁸R⁹,
wherein
R⁵ and R⁶ are co-ordinated such that either both radicals are acid or basic or one of the radicals is neutral,
R⁷ and R⁸ independently of one another are
H or alkyl having 1-5 C atoms,
and
R⁹ is alkyl having ¹⁻⁵ C atoms

10. Polymeric sorbent according to one of Claims 1 to 5, characterized in that the polymerized-on polymer contains monomer units of the formula IV 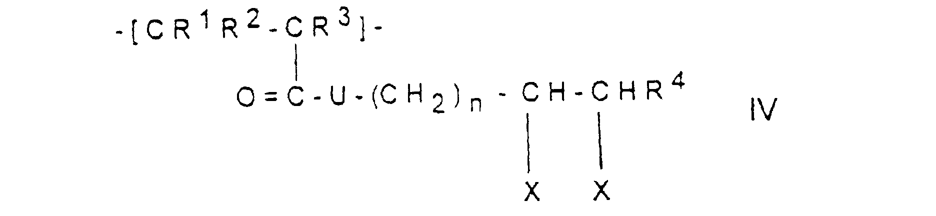 wherein
R¹, R² and R³ independently of one another are
H or CH₃,
R⁴ is H, alkyl having 1-5 C atoms or aryl having 6-12 C atoms,
U is -O- or -NH-,
n is an integer between 1 and 5 and
one radical X is a separation effector and the other radical X is OH.

11. Polymeric sorbent according to Claim 10, characterized in that the separation effector is an ionic group chosen from -PO₄H₂, -SO₃H, -NR⁷R⁸ or -N⁺R⁷R⁸R⁹,
wherein
R⁷ and R⁸ independently of one another are
H or alkyl having 1-5 C atoms
and
R⁹ is alkyl having 1-5 C atoms
with the proviso that if X = -N⁺R⁷R⁸R⁹, R⁷ and R⁸ cannot be H.

12. Polymeric sorbent according to Claim 10, characterized in that the separation effector is a hydrophobic grouping -OR¹⁰ or -NHR¹⁰,
wherein
R¹⁰ is C₁-C₂₀-alkyl, C₆-C₂₅-aryl, C₇-C₂₅-alkylaryl or C₇-C₂₅-arylalkyl, and wherein these radicals can also be derivatized with nitrile or C₁-C₅-alkoxy, and wherein also one or more non-adjacent CH₂ groups can be replaced by NH or O, or also one or more CH groups can be replaced by N.

13. Polymeric sorbent according to Claim 10, characterized in that the separation effector is a metal chelate affinity group.

14. Polymeric sorbent according to Claim 10, characterized in that the separation effector is a thiophilic radical.

15. Affinity support which can be prepared from a polymeric sorbent according to one of Claims 1-9 or 11.

16. Immobilized enzyme which can be prepared from a polymeric adsorbent according to one of Claims 1-9 or 11.

17. Shaped article consisting essentially of a polymeric adsorbent according to one of Claims 1 to 14.

18. Shaped article on which an affinity ligand or an enzyme is immobilized, which can be prepared from a polymeric adsorbent according to one of Claims 1-9 or 11.

19. Process for the chromatographic separation of substances, characterized in that a polymeric sorbent according to one of Claims 1 to 15 is used.

## Revendications

1. Polymère absorbant produit par les étapes de réaction suivantes :
a) introduction de doubles liaisons polymérisables par la réaction d'un groupe amino d'un polyamide avec un composé amino-réactif qui contient une double liaison polymérisable, ou par la réaction des groupes carboxyles d'un polyamide avec un composé réagissant avec les groupes carboxyles qui contient une double liaison polymérisable, à la condition que ladite liaison amino-réactive ne contienne pas un cycle oxiran lorsque le polymère de base réagit, sans avoir été mis en réaction auparavant avec une liaison diamino.
b) polymérisation des monomères sur le polyamide dérivé de l'étape a), dans laquelle lesdits monomères contiennent un groupe époxy, un groupe azlactone ou un groupe précurseur d'un groupe azlactone ou un effecteur de séparation.
c) mise en réaction optionnelle desdits groupes époxy ou azlactone dans un effecteur de séparation.

2. Polymère absorbant selon la revendication 1, caractérisé en ce que avant la réaction avec le composé amino-réactif de l'étape a) des groupes carboxyles du polymère réagissent avec un composé diamino.

3. Polymère absorbant selon la revendication 1, caractérisé en ce que avant la réaction avec le composé qui contient des groupes carboxyles de l'étape a) on fait réagir les groupes amino du polymère avec un acide dicarboxylique ou un anhydride d'un acide dicarboxylique.

4. Polymère absorbant selon la revendication 1 ou 2, dans lequel au cours de l'étape a) on fait réagir les groupes amino du polyamide avec un anhydride d'acide carboxylique éthylénique insaturé.

5. Polymère absorbant selon la revendication 1 ou 2, dans lequel au cours de l'étape a) les groupes amino du polyamide réagissent avec un dérivé de vinylazlactone de formule I : 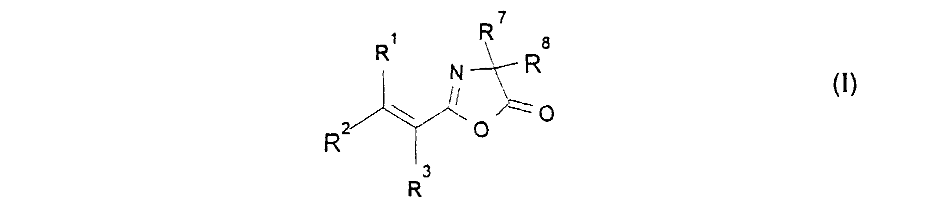 dans laquelle
R¹, R², et R³ représentent indépendamment les uns des autres
H ou CH₃;
R⁷ et R⁸ représentent indépendamment l'un de l'autre
H ou un alkyle C₁- C₅.

6. Polymère absorbant selon l'une des revendications 1 à 5, caractérisé en ce qu'on utilise comme monomère de l'acide acrylique dans l'étape de réaction b).

7. Polymère absorbant selon l'une des revendications 1 à 5, caractérisé en ce que le polymère polymérisé contient des unités monomères de formule II : 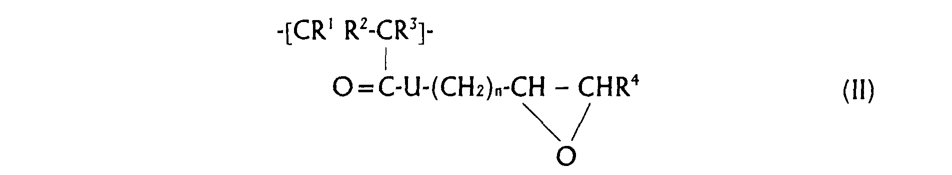 dans laquelle
R¹, R², et R³ représentent indépendamment les uns des autres H ou CH₃
R⁴ représente H, un alkyle avec 1 - 5 atomes de C ou un aryle avec 6 - 12 atomes de C,
U représente -O- ou -NH- et
n représente un nombre entier compris entre 1 et 5.

8. Polymère absorbant selon l'une des revendications 1 à 5, caractérisé en ce que le polymère polymérisé contient des unités monomères de formule V, 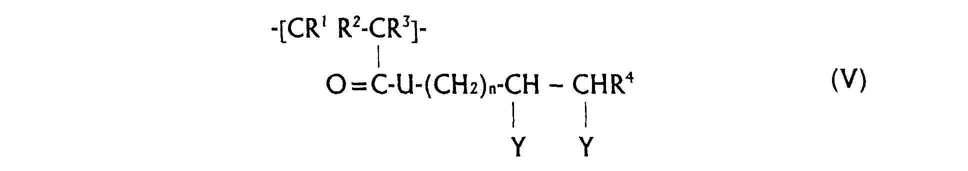 dans laquelle
R¹, R² et R³ représentent indépendamment les uns des autres H ou CH3
R⁴ représente H, un alkyle avec 1 - 5 atomes de C ou un aryle 6 - 12 atomes de C
U représente -O- ou -NH-
et n représente un nombre entier compris entre 1 et 5,
un résidu Y représente un résidu de formule VI et l'autre résidu Y représente OH 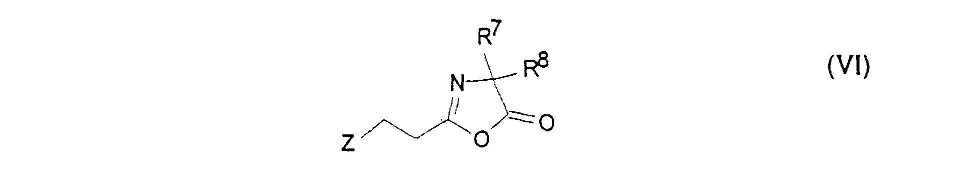
Z représente -S ou -NH et
R⁷ et R⁸ représentent indépendamment l'un de l'autre H, ou un alkyle à 1-5 atomes de C.

9. Polymère absorbant selon l'une des revendications 1 à 5, caractérisé en ce que le polymère polymérisé contient des unités monomères de formule III : 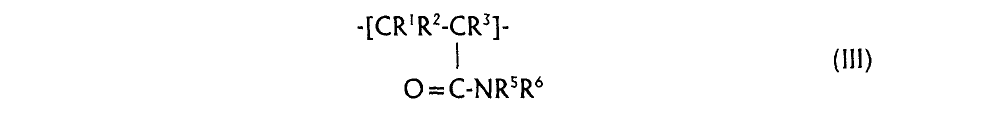 dans laquelle
R¹, R² et R³ représentent indépendamment les uns des autres H ou CH₃
R⁵ représente H, un alkyle à 1-5 atomes de C substitué avec -COOH, avec SO₃H, avec -NR⁷R⁸ ou avec -N⁺R⁷R⁸R⁹, un aryle à 6-12 atomes de carbone substitué avec -COOH, avec SO₃H, avec -NR⁷R⁸ ou avec -N⁺ R⁷R⁸R⁹
R⁶ représente un alkyle à 1-5 atomes de C substitué avec -COOH, avec SO₃H, avec -NR⁷R⁸ ou avec ⁻N⁺R⁷R⁸R⁹, ou un aryle à 6-12 atomes de carbone substitué avec -COOH, -SO₃H, avec -NR⁷R⁸ ou -N⁺ R⁷R⁸R⁹
dans laquelle R⁵ et R⁶ sont assortis de manière à ce que soit les deux résidus soient acides ou basiques soit un des résidus est neutre,
R⁷ et R⁸ représentent indépendamment l'un de l'autre H, un alkyle à 1-5 atomes de C et
R⁹ représente un alkyle à 1-5 atomes de C.

10. Polymère absorbant selon l'une des revendications 1 à 5, caractérisé en ce que le polymère polymérisé contient des unités monomères de formule IV, 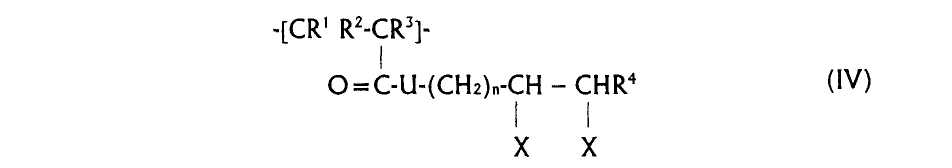 dans laquelle
R¹, R² et R³ représentent indépendamment les uns des autres H ou CH3
R⁴ représente H, un alkyle à 1-5 atomes de C ou un aryle à 6-12 atomes de C
U représente -O- ou -NH-
n représente un nombre entier compris entre 1 et 5,
un résidu X représente un effecteur de séparation, de même que l'autre résidu X représente OH.

11. Polymère absorbant selon la revendication 10, caractérisé en ce que l'effecteur de séparation présente un groupe ionique, choisi dans le groupe constitué par -PO₄H₂, -SO₃H, -NR⁷R⁸ ou -N⁺R⁷R⁸R⁹, dans lesquels R⁷ et R⁸ représentent indépendamment l'un de l'autre H, un alkyle à 1-5 atomes de C et R⁹ représente un alkyle à 1-5 atomes de C à la condition que lorsque X = -N⁺ R⁷R⁸R⁹, R⁷ et R⁸ ne peuvent être H.

12. Polymère absorbant selon la revendication 10, caractérisé en ce que l'effecteur de séparation représente un groupe hydrophobe -OR¹⁰ ou -NHR¹⁰ dans lequel R¹⁰ représente un alkyle C₁-C₂₀, un aryle C₆-C₂₅, un alkylaryle C₇-C₂₅ ou un arylalkyle C₇-C₂₅, et dans lequel ces résidus peuvent également être dérivés avec un nitrile ou un alkoxy C₁-C₅, et dans lequel également un ou plusieurs groupes CH2 non voisins peuvent être remplacés par NH ou O ou bien un ou plusieurs groupes CH peuvent être remplacés par N.

13. Polymère absorbant selon la revendication 10, caractérisé en ce que l'effecteur de séparation est un groupe d'affinité-métalchélate.

14. Polymère absorbant selon la revendication 10, caractérisé en ce que l'effecteur de séparation présente un résidu thiophile.

15. Support d'affinité produit à partir du polymère absorbant selon l'une des revendications 1-9 ou 11.

16. Enzyme immobilisé produit à partir d'un polymère absorbant selon l'une des revendications 1-9 ou 11.

17. Article façonné constitué majoritairement d'un polymère absorbant selon l'une des revendications 1 à 14.

18. Article façonné, sur lequel un ligand d'affinité ou un enzyme est immobilisé, produit à partir d'un polymère absorbant selon l'une des revendications 1 à 9 ou 11.

19. Procédé de séparation chromatographique de substances, caractérisé en ce que l'on utilise un polymère absorbant selon l'une des revendications 1 à 15.
